# EUROPÄISCHE PATENTSCHRIFT

(11) **EP 1 325 718 B1**
(45) Veröffentlichungstag und Bekanntmachung des Hinweises auf die Patenterteilung: **21.09.2005**
(21) Anmeldenummer: 03000062.4
(22) Anmeldetag: 07.01.2003
(51) Int. Cl.: A61F 2/38

(54) **Tibiakomponente einer Kniegelenkendoprothese**
Tibial element for a replacement knee prosthesis
Elément tibial pour une prothèse de remplacement du genou

(30) Priorität: 07.01.2002 DE 10200263
(43) Veröffentlichungstag der Anmeldung: 09.07.2003
(73) Patentinhaber: Plus Orthopedics AG, 6343 Rotkreuz (CH)
(72) Erfinder: Brack, René, 6330 Cham (CH)
(74) Vertreter: Popp, Eugen

(56) Entgegenhaltungen:
- EP-A- 0 864 306
- GB-A- 2 345 446
- US-A- 6 123 728

## Beschreibung

Die Erfindung betrifft eine Tibiakomponente einer Kniegelenkendoprothese gemäß dem Oberbegriff des Anspruches 1.

Eine derartige Tibiakomponente ist bekannt aus der EP 0 864 306 Al oder WO 99/13804.

Diesen bekannten Tibiakomponenten ist gemeinsam, daß die am Lagerzapfen sich radial nach außen erstreckenden Vorsprünge am freien stirnseitigen Ende des Lagerzapfens ausgebildet sind. Aufgrund dieser Konstruktion ist zur Herstellung nur einer Rotation des Lagerkörpers auf dem zugeordneten Tibiaplateau eine gesonderte Fixierschraube entsprechend dem Vorschlag nach der WO 99/13804 erforderlich, um die ursprünglich noch mögliche Translationsbewegung des Lagerkörpers auf dem Tibiaplateau zu blockieren. Bei dem Vorschlag nach der WO 99/13804 wird die erwähnte Fixierschraube durch den Lagerkörper hindurch stirnseitig in den erwähnten Lagerzapfen eingeschraubt. Zur Blockierung einer Translationsbewegung ist nach dem Stand der Technik also ein gesondertes Bauteil erforderlich, wodurch der Konstruktionsaufwand insgesamt nicht unerheblich komplizierter und damit entsprechend teurer wird. Außerdem ist beim Stand nach der WO 99/13804 nachteilig, daß die Fixierschraube durch den Lagerkörper hindurch, d.h. von der Femurseite her in den Lagerkörper eingeschraubt werden muß. Dadurch erfährt der Lagerkörper eine nicht unerhebliche Schwächung. Auch ist eine Migration von Abriebpartikeln durch den Lagerkörper hindurch sowohl von der Tibiaseite zur Femurseite als auch von der Femurseite zur Tibiaseite hin möglich.

Ferner beschreibt die GB 2 345 446 A eine Knieprothese, die eine Tibia- und eine Femurkomponente sowie zwei Meniskuskomponenten umfaßt. Eine der Meniskuskomponenten wirkt mit der Tibiakomponente so zusammen, daß eine Relativbewegung zwischen beiden vollständig vermieden wird. Die andere Meniskuskomponente ist so konzipiert, daß eine begrenzte Relativbewegung zwischen ihr und der Tibiakomponente möglich ist. Die Tibiakomponente weist dort zwei Zapfen auf, die jeweils einen ringförmigen Vorsprung aufweisen, die in entsprechende Hinterschneidungen der Meniskuskomponente einrastbar sind (snap-fit manner).

Nachteilig bei diesem Stand der Technik ist die aufwendige Ausbildung von zwei Zapfen, die in zwei entsprechend aufwendig herzustellende Ausnehmungen der Meniskuskomponente eingreifen. Durch diese Zwei-Zapfen-Ausführungsform ist des weiteren die Flexibilität der bekannten Knieprothese stark eingeschränkt; die erlaubt praktisch auch keine Rotation. Des weiteren haben die zwei Zapfen einen hohen Reib- bzw. Gleitwiderstand zur Folge, der einerseits zu Abrieb und andererseits zu einem erhöhten notwendigen Kraftaufwand führt.

Der vorliegenden Erfindung liegt die Aufgabe zugrunde, eine Tibiakomponente der eingangs genannten Art zu schaffen, die ohne zusätzliche Bauteile dem Operateur die Möglichkeit läßt, für den Lagerkörper entweder nur eine Rotation oder alternativ sowohl eine Rotation als auch Translation vorzusehen.

Diese Aufgabe wird erfindungsgemäß durch eine Tibiakomponente gemäß Patentanspruch 1 gelöst, wobei wenigstens zwei Zapfenorsprünge vorgesehen sind, die entweder diametral oder in einem vorbestimmten Winkel (α) zueinander stehen.

Damit erlaubt der Lagerzapfen bei entsprechender Ausbildung der zugeordneten Zapfenaufnahme an der Unterseite des Lagerkörpers entweder nur eine Rotation desselben auf der Tibialagerfläche oder alternativ sowohl eine Rotation als auch Translation. Zur Herstellung nur einer Rotation des Lagerkörpers auf der Tibialagerfläche ist ein gesondertes Bauteil nicht erforderlich. Es braucht lediglich die Zapfenaufnahme einen geteilten Querschnitt aufweisen derart, daß der Querschnitt bis zur Hinterschneidung oder Zapfenvorsprung-Aufnahmenut dem Öffnungsquerschnitt entspricht, während der Querschnitt im übrigen kreiszylindrisch ist entsprechend dem Querschnitt des kreiszylindrischen Lagerzapfens. Dem letztgenannten Querschnitt ist der kreiszylindrische Fortsatz des Lagerzapfens über den wenigstens einen sich radial nach außen erstreckenden Vorsprung hinaus zugeordnet.

Vorzugsweise sind die Zapfenvorsprünge jeweils stiftartig ausgebildet. Sie können jedoch genauso gut flansch- oder scheibenartig ausgebildet sein, wobei in letztgenanntem Fall die Vorsprünge durch jeweils mondsichelartige Scheiben definiert sind.

Bei wenigstens zwei Zapfenvorsprüngen sind diese entweder diametral relativ zur Zapfenlängsachse oder in einem vorbestimmten Winkel, insbesondere einem Winkel von etwa 90° zueinander gerichtet.

Zur Montage des Lagerkörpers weist die Zapfenaufnahme im Lagerkörper einen Öffnungsquerschnitt auf, der der Projektionsfläche des Lagerzapfens samt Vorsprung bzw. Vorsprüngen entspricht, d.h. der Projektion des Lagerzapfens in stirnseitiger Draufsicht. Damit kann der Lagerzapfen samt Vorsprung bzw. Vorsprüngen in einer vorbestimmten Rotationsstellung zwischen Lagerkörper und Tibialagerfläche in die Zapfenaufnahme eingefügt werden, und zwar soweit, bis der bzw. die Zapfenvorsprünge bei Drehung des Lagerkörpers auf der Tibialagerfläche in eine relative Ausgangsstellung die in der Zapfenaufnahme ausgebildete Hinterschneidung unter - bzw. in die in der Zapfenaufnahme ausgebildete Nut eingreifen. Die relative Ausgangsstellung zwischen Lagerkörper und Tibialagerfläche ist diejenige Stellung, in der die femurseitig im Lagerkörper ausgebildeten Lagerschalen posterior/anterior ausgerichtet sind.

Vorzugsweise befindet sich der wenigstens eine Zapfenvorsprung etwa auf Höhe von etwa 2/3 der Gesamthöhe des Lagerzapfens. Bei einer Gesamthöhe des Lagerzapfens beispielsweise 8 mm, befindet sich der wenigstens eine sich radial nach außen erstreckende Zapfenvorsprung auf einer Höhe von etwa 5 mm über der Tibialagerfläche. Die Höhe bzw. Dicke des oder der Zapfenvorsprünge beträgt dann etwa 1 mm, so daß der Zapfen über die Zapfenvorsprünge hinaus um etwa 2 mm fortgesetzt ist. Ganz wesentlich für die Verwirklichung des Erfindungsgedankens ist, daß die erfindungsgemäße Tibiakomponente mit einem Lagerkörper kombinierbar ist, dessen Zapfenaufnahme entweder durchgehend einen Querschnitt entsprechend dem Öffnungsquerschnitt der Zapfenaufnahme oder einen über die Tiefe der Zapfenaufnahme geteilten Querschnitt aufweist derart, daß der Querschnitt bis zur Hinterschneidung oder Zapfenvorsprung-Aufnahmenut dem Öffnungsquerschnitt entspricht, während der Querschnitt im übrigen kreiszylindrisch ist entsprechend dem Querschnitt des kreiszylindrischen Lagerzapfens. Der erstgenannte Lagerkörper erlaubt eine Fixierung desselben auf der Tibiagleitfläche so, daß der Lagerkörper auf der Tibiagleitfläche sowohl drehbar als auch translatorisch hin- und herbewegbar ist. Die zweite Ausführungsform des Lagerkörpers erlaubt nur eine Rotation desselben auf der Tibiagleitfläche, ohne daß es eines gesonderten Bauteils zur Blockierung der Translationsbewegung bedarf.

Eine besonders vorteilhafte Ausführungsform zeichnet sich dadurch aus, daß der oder die Zapfenvorsprünge sich in Richtung posterior/anterior erstrecken, während der korrespondierende Öffnungsquerschnitt der Zapfenaufnahme im Lagerkörper sich in einem Winkel, insbesondere in einem Winkel von etwa 45° dazu erstreckt. Bei dieser Ausführungsform ist das Aufstecken des Lagerkörpers auf den Aufnahmezapfen ohne größere Behinderung durch die Kreuzbänder etc. möglich. Gleichzeitig ist eine große Sicherheit gegen eine Luxation des Lagerkörpers gegeben, da im Normalfall eine Rotation des Lagerkörpers um 45°, d.h. in eine Stellung, in der der Lagerzapfen theoretisch aus der zugeordneten Aufnahmeöffnung herausgleiten könnte, nicht möglich erscheint.

Im Extremfall erstreckt sich die Aufnahmeöffnung an der Unterseite des Lagerkörpers quer zur anterior/posterior-Achse, d.h. in Richtung etwa parallel zur Längserstreckung des Lagerkörpers bzw. in Richtung medial/lateral. Bei dieser Ausführungsform würden jedoch die Kreuzbänder beim Aufstecken des Lagerkörpers nicht unerheblich belastet werden, da der Lagerkörper beim Aufstecken auf den Lagerzapfen sich von hinten (posterior) nach vorne (anterior) erstreckt, d.h. quer zur Tibiagleitfläche.

Nachstehend werden bevorzugte Ausführungsbeispiele einer erfindungsgemäßen Tibiakomponente in Zuordnung zu geeigneten Lagerkörpern anhand der beigefügten Zeichnungen erläutert. Diese zeigen in:
- Figur 1: eine erste Ausführungsform einer erfindungsgemäßen Tibiakomponente in Ansicht von anterior;
- Figur 1a: den Lagerkörper gemäß Figur 1 in Unteransicht;
- Figur 1b: das den Lagerzapfen umfassende Teil (Tibiaplateau) der in Figur 1 dargestellten Tibiakomponente in perspektivischer Ansicht von schräg oben;
- Figur 2: die Tibiakomponente gemäß Figur 1 in Draufsicht;
- Figur 3: die Tibiakomponente gemäß Figur 1 ebenfalls in Draufsicht, wobei sich der Lagerkörper in einer Rotationsstellung befindet;
- Figur 4: eine zweite Ausführungsform einer erfindungsgemäßen Tibiakomponente in Ansicht von anterior;
- Figur 4a: den Lagerkörper gemäß Figur 4 in Unteransicht;
- Figur 5: die Tibiakomponente gemäß Figur 4 in Draufsicht;
- Figur 6: die Tibiakomponente gemäß Figur 4 in Draufsicht, wobei der Lagerkörper um 45° rotiert ist, und zwar in eine Stellung, in der der Lagerkörper auf den Lagerzapfen aufsteckbar bzw. vom Lagerzapfen abziehbar ist;
- Figur 7: einen Schnitt durch die Tibiakomponente gemäß Figur 1 Längslinie A - A;
- Figur 8: einen Schnitt durch die Tibiakomponente gemäß Figur 4 Längslinie B - B;
- Figur 9-11: eine weitere Ausführungsform einer Tibiakomponente in Draufsicht unter Darstellung verschiedener Relativstellungen zwischen Lagerkörper und dem die Tibialagerfläche umfassenden Bauteil (Tibiaplateau).

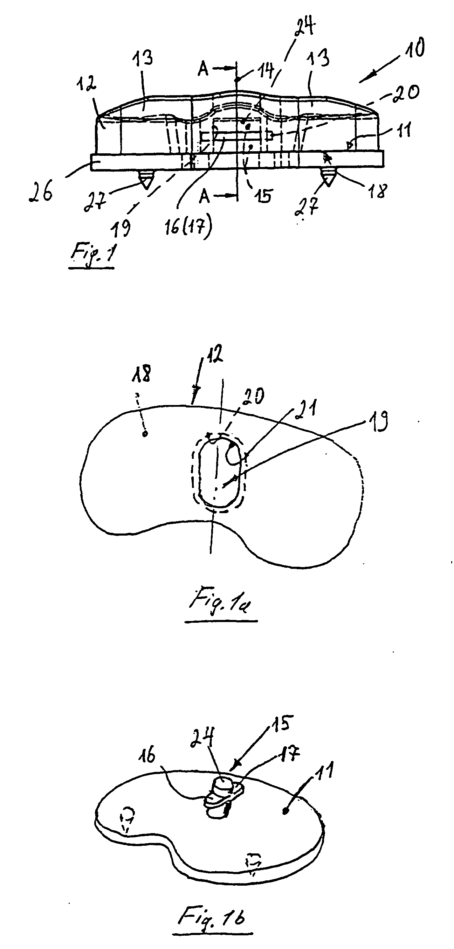

Die in den Figuren 1-3 dargestellte Ausführungsform einer Tibiakomponente 10 einer im übrigen nicht näher dargestellten Kniegelenkendoprothese definiert eine ebene Tibialagerfläche 11 an der Femurseite des zugeordneten Bauteils bzw. sog. Tibiaplateaus 26. Auf der Tibialagerfläche ist ein Lagerkörper 12 aus humanverträglichem Kunststoff, insbesondere Polyethylen (PE) gleitend gelagert. An der Femurseite des Lagerkörpers 12 sind zwei konkav gewölbte Lagerschalen 13 zur beweglichen Aufnahme einer Femurkomponente ausgebildet. Die dargestellte Tibiakomponente weist eine Drehführung für den Lagerkörper 12 auf. Diese Drehführung gewährleistet eine Führung des Lagerkörpers auf der Tibialagerfläche 11 um eine senkrecht auf dieser stehenden Drehachse 14, wobei sie einen aufrecht auf der Tibialagerfläche 11 stehenden kreiszylindrischen Lagerzapfen 15 mit zwei sich radial nach außen erstreckenden Vorsprüngen 16, 17 umfaßt. Der so ausgebildete Lagerzapfen 15 ist in eine an der der Tibialagerfläche 11 zugekehrten Gleitfläche 18 des Lagerkörpers 12 bzw. in eine an der Unterseite desselben ausgebildeten Zapfenaufnahme 19 mit den Zapfenvorsprüngen 16, 17 korrespondierender Umfangsnut 20 einfügbar. Die Umfangsnut 20 innerhalb der Zapfenaufnahme 19 ist also zur Aufnahme der beiden Zapfenvorsprünge 16, 17 ausgebildet. Die Zapfenvorsprünge 16, 17 sind jeweils mondsichelartige Scheiben und erstrecken sich diametral zur Zapfenlängsachse nach posterior bzw. anterior. Die Zapfenlängsachse definiert die bereits erwähnte Drehachse 14 des Lagerkörpers 12.

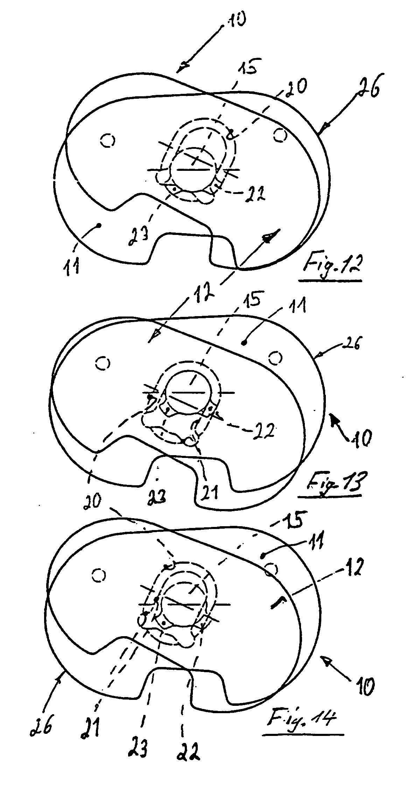

Wie insbesondere die Figuren 1b und 7 sehr deutlich erkennen lassen, erstrecken sich die Zapfenvorsprünge 16, 17 im vorbestimmten Abstand vom freien Zapfenende 24. Sie liegen auf Höhe von etwa 2/3 der Gesamthöhe des Lagerzapfens 15.

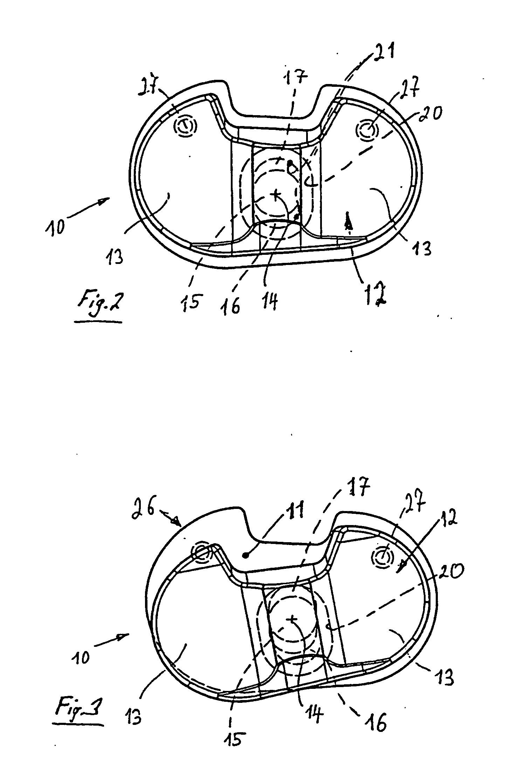

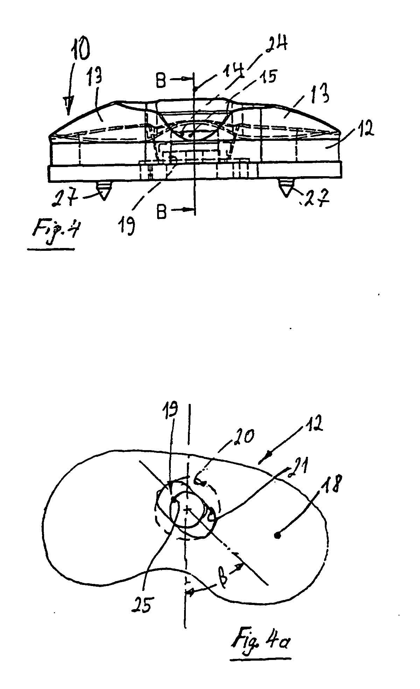

Die Zapfenaufnahme 19 im Lagerkörper 12 weist einen Öffnungsquerschnitt 21 auf, der der längsovalen Projektionsfläche des Lagerzapfens 15 samt Vorsprüngen 16, 17 entspricht, so daß der Lagerzapfens samt Vorsprüngen in vorbestimmter Rotationsstellung zwischen Lagerkörper und Tibialagerfläche bzw. Tibiaplateau in die Zapfenaufnahme einfügbar ist, und zwar soweit, bis die Zapfenvorsprünge bei Drehung des Lagerkörpers auf der Tibialagerfläche 11 in die in der Zapfenaufnahme 19 ausgebildete Nut 20 eingreifen. Der Eingriff der Zapfenvorsprünge 16, 17 in die Umfangsnut 20 innerhalb der Zapfenaufnahme 19 läßt sich insbesondere Figur 3 sehr gut entnehmen. Entsprechend Figur 1a und Figur 2 erstreckt sich die längsovale Aufnahmeöffnung der Zapfenaufnahme 19 in Richtung posterior/anterior. Da sich auch die Vorsprünge 16, 17 am Zapfen 15 in Richtung posterior/anterior erstrecken, läßt sich der Lagerkörper 12 in einer entsprechenden Relativstellung gemäß Figur 2 auf den Lagerzapfen 15 samt Vorsprüngen 16, 17 aufstecken. Beim Aufstecken des Lagerkörpers 12 muß also darauf geachtet werden, daß sich die längsovale Aufnahmeöffnung der Zapfenaufnahme 19 ebenfalls in Richtung posterior/anterior erstreckt.

Wie den Figuren 2 und 3 sehr gut entnommen werden kann, zeichnet sich die beschriebene Ausführungsform einer Tibiakomponente dadurch aus, daß der Lagerkörper 12 auf der Tibialagerfläche 11 sowohl drehbar als auch translatorisch verschiebbar gelagert ist.

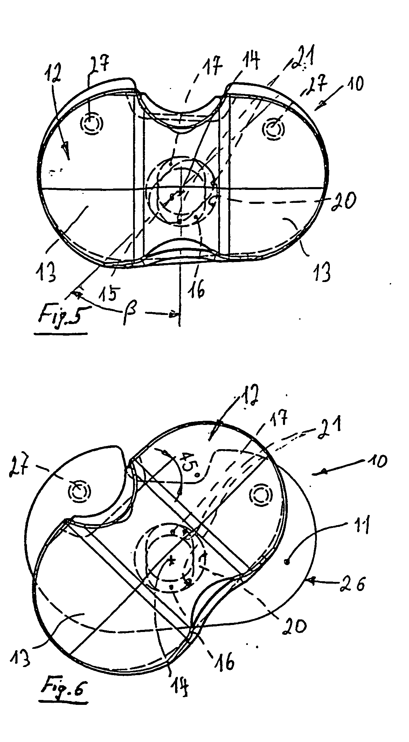

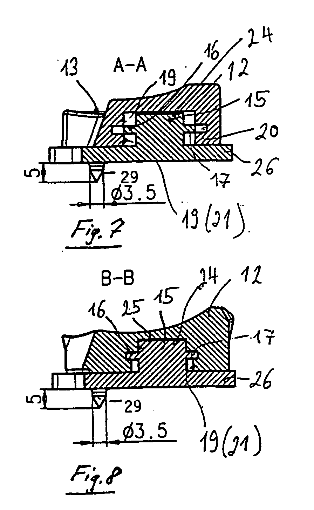

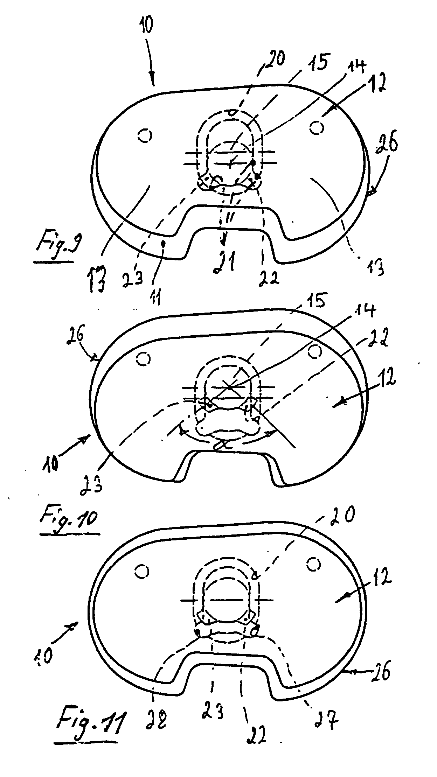

Um ein ungewolltes Abheben des Lagerkörpers 12 von der Tibialagerfläche 11 bei Normalstellung des Lagerkörpers 12 entsprechend Figur 2 zu vermeiden, ist es vorteilhaft, wenn sich die längsovale Aufnahmeöffnung der Zapfenaufnahme 19 an der Unterseite des Lagerkörpers 12 in einem Winkel zur posterioren/anterioren Erstreckung der zugeordneten Zapfenvorsprünge 16, 17 erstreckt, so wie dies bei dem Ausführungsbeispiel nach den Figuren 4 bis 6 und 8 der Fall ist. Wie Figur 6 entnommen werden kann, erstrecken sich die Zapfenvorsprünge 16, 17 in Richtung posterior/anterior ebenso wie bei der Ausführungsform nach den Figuren 1 bis 3 und 7, während der korrespondierende längsovale Öffnungsquerschnitt 21 der Zapfenaufnahme 19 im Lagerkörper 12 sich in einem Winkel β von 45° dazu erstreckt. Aus diesem Grunde ist auch nur in dieser 45°-Stellung des Lagerkörpers relativ zur Tibialagerfläche 11 bzw. zum Tibiaplateau 26 entsprechend Figur 6 eine Montage des Lagerkörpers 12 auf dem Tibiaplateau 26 möglich. Beim Zurückdrehen des Lagerkörpers 12 in Normalstellung entsprechend Figur 5 greifen die Zapfenvorsprünge 16, 17 in die Umfangsnut 20 innerhalb der Zapfenaufnahme 19 ein. Bei der Ausführungsform nach den Figuren 4 bis 6 und 8 ist die innerhalb der Zapfenaufnahme 19 ausgebildete Umfangsnut 20 kreisförmig, während sie bei dem ersten Ausführungsbeispiel der Kontur des längsovalen Öffnungsquerschnitts 21 der Zapfenaufnahme 19 folgt. Die kreisförmige Aufnahmenut 20 entsprechend den Figuren 4 bis 6 und 8 ist herstellungstechnisch einfacher als die anhand des ersten Ausführungsbeispieles dargestellte längsovale Aufnahmenut.

Die zweite Ausführungsform, bei der gleiche Teile mit denselben Bezugsziffern wie bei der ersten Ausführungsform gekennzeichnet sind, unterscheidet sich von der ersten Ausführungsform insbesondere noch dadurch, daß der Querschnitt der Zapfenaufnahme 19 zweigeteilt ist. Der Querschnitt der Zapfenaufnahme 19 entspricht bis zur Zapfenvorsprung-Aufnahmenut 20 dem Öffnungsquerschnitt 21. Im übrigen ist der Querschnitt 25 der Zapfenaufnahme 19 kreiszylindrisch ausgebildet, und zwar entsprechend dem Querschnitt des kreiszylindrischen Lagerzapfens 15. Dementsprechend ist der sich über die Lagervorsprünge 16, 17 hinauserstreckende Abschnitt des Lagerzapfens 15 in diesen letztgenannten Querschnitt so einpaßbar, daß der Lagerkörper 12 lediglich eine Rotationsbewegung ausführen kann.

Das Tibiaplateau beider Ausführungsformen ist im übrigen noch dadurch gekennzeichnet, daß dieses an der Unterseite zwei Knochen-Fixierdorne 29 aufweist, und zwar nahe der posterioren Begrenzung des Tibiaplateaus 26.

Der Zapfen 15 ist mit dem Tibiaplateau 26 einstückig verbunden. Grundsätzlich ist es jedoch auch denkbar, den Lagerzapfen 15 als gesondertes Bauteil auszubilden und mittels einer Befestigungsschraube auf dem Tibiaplateau 26 zu fixieren.

Zum zweiten Ausführungsbeispiel sei auch noch besonders auf Figur 4a hingewiesen, die den Lagerkörper 12 von der Unterseite bzw. seiner tibiaseitigen Gleitfläche 18 her zeigt. Es ist deutlich die Ausrichtung der längsovalen Aufnahmeöffnung 21 der Zapfenaufnahme 19 erkennbar ebenso wie die kreisförmige Zapfenvorsprung-Aufnahmenut 20. Auch ist sehr gut erkennbar der Kreisquerschnitt 25 entsprechend dem Querschnitt des kreiszylindrischen Lagerzapfens 15 im unteren Bodenbereich der Zapfenaufnahme 19. Dieser kreiszylindrische Querschnitt 25 stellt sicher, daß der Lagerkörper 12 um den Zapfen 15 bzw. dessen Längsachse 14 lediglich eine Rotationsbewegung ausführen kann.

Bei der dritten Ausführungsform entsprechend den Figuren 9 bis 14 sind ebenfalls wieder gleiche Teile mit denselben Bezugsziffern gekennzeichnet wie bei den vorangehenden Ausführungsformen. Auf eine entsprechende Beschreibung dieser Teile wird daher an dieser Stelle verzichtet. Es wird diesbezüglich auf die Beschreibung der beiden ersten Ausführungsformen verwiesen.

Die dritte Ausführungsform ist insbesondere dadurch gekennzeichnet, daß der Lagerzapfen 15 zwei stiftartige Vorsprünge 22, 23 aufweist, die in einem vorbestimmten Winkel α von hier etwa 100° zueinander stehen (siehe Figur 10).

Entsprechend diesen Vorsprüngen muß auch der Öffnungsquerschnitt 21 der Zapfenaufnahme 19 im Lagerkörper 12 Ausnehmungen aufweisen, die zum Beispiel in Figur 11 mit den Bezugsziffern 27, 28 gekennzeichnet sind. Bei entsprechender Ausrichtung dieser Ausnehmungen 27, 28 zu den stift- bzw. nasenartigen Vorsprüngen 22, 23 läßt sich der Lagerkörper 12 auf den Lagerzapfen 15 aufstecken. Diese Relativstellung zwischen Lagerkörper 12 und Tibialagerfläche 11 bzw. Tibiaplateau 26 ist in Figur 9 dargestellt.

Alle anderen Figuren 10 bis 14 zeigen unterschiedliche Relativstellungen zwischen Lagerzapfen 15 samt Vorsprüngen 22, 23 und Lagerkörper 12, wobei erkennbar ist, daß in all diesen anderen Relativstellungen die Zapfenvorsprünge 22, 23 in die längsovale Umfangsnut 20 innerhalb der Zapfenaufnahme 19 eingreifen und somit ein Abheben des Lagerkörpers 12 vom Tibiaplateau 26 verhindern.

Bei der dargestellten Ausführungsform sind die nasen- bzw. stiftartigen Zapfenvorsprünge 22, 23 nach posterior gerichtet. Es ist jedoch jede andere Ausrichtung ebenso gut denkbar. Nur in der Praxis hat sich die dargestellte Ausrichtung der Zapfenvorsprünge 22, 23 bewährt, da nach dem Aufstecken des Lagerkörpers 12 in einer Position entsprechend Figur 9 dieser sich bedingt durch die Anatomie in eine Position verschiebt, in der die Zapfenvorsprünge 22, 23 in die zugeordnete Aufnahmenut 20 eingreifen, d.h. zumindest in eine Position entsprechend Figur 11. Bei einer nur geringfügigen Rotation des Lagerkörpers 12 erfolgt ebenfalls der erwähnte Eingriff wenigstens eines der beiden Zapfenvorsprünge 22, 23 in die Umfangsnut 20, wie die Figuren 12 bis 14 erkennen lassen. In all diesen Fällen ist der Lagerkörper 20 sicher auf der Tibialagerfläche 11 bzw. dem Tibiaplateau 26 gesichert.

Auch bei der Ausführungsform nach den Figuren 9 bis 14 ist es denkbar, den Querschnitt der Zapfenaufnahme 19 zweigeteilt auszubilden, d.h. den bodenseitigen Abschnitt der Zapfenaufnahme 19 mit einem kreiszylindrischen Querschnitt entsprechend dem kreiszylindrischen Querschnitt des Lagerzapfens 15 zu versehen. In diesen Querschnitt greift dann der sich über die Zapfenvorsprünge 22, 23 hinauserstreckende Abschnitt des Lagerzapfens 15 ein.

Das Tibiaplateau samt Lagerzapfens ist in herkömmlicher Weise aus einem humanverträglichen Material hergestellt, insbesondere einer Titanlegierung.

Sämtliche in den Anmeldungsunterlagen offenbarten Merkmale werden als erfindungswesentlich beansprucht, soweit sie einzeln oder in Kombination gegenüber dem Stand der Technik neu sind.

### Bezugszeichenliste

- 10: Tibiakomponente
- 11: Tibialagerfläche
- 12: Lagerkörper
- 13: Lagerschale
- 14: Drehachse
- 15: Lagerzapfen
- 16: radialer Vorsprung
- 17: radialer Vorsprung
- 18: Gleitfläche bzw. Unterseite des Lagerkörpers
- 19: Zapfenaufnahme
- 20: Zapfenvorsprung-Aufnahmenut
- 21: Öffnungsquerschnitt
- 22: radialer Vorsprung
- 23: radialer Vorsprung
- 24: freies Zapfenende
- 25: Querschnitt
- 26: Tibiaplateau
- 27: Ausnehmung im Öffnungsquerschnitt
- 28: Ausnehmung im Öffnungsquerschnitt
- 29: Knochen-Fixierdorn

## Patentansprüche

1. Tibiakomponente (10) einer Kniegelenkendoprothese mit einer ebenen Tibialagerfläche (11), einem auf dieser gleitend gelagerten Lagerkörper (12) mit zwei konkav gewölbten Lagerschalen (13) zur beweglichen Aufnahme einer Femurkomponente, und mit einer Drehführung, die den Lagerkörper (12) auf der Tibialagerfläche (11) um eine senkrecht auf dieser stehenden Drehachse (14) führt, wobei die Drehführung einen aufrecht auf der Tibialagerfläche (11) stehenden kreiszylindrischen Lagerzapfen (15) mit wenigstens einem sich radial nach außen erstreckenden Vorsprung (16, 17; 22, 23) umfaßt, der in eine an der der Tibialagerfläche (11) zugekehrten Gleitfläche (18) des Lagerkörpers (12) bzw. in eine an dessen Unterseite ausgebildeten Zapfenaufnahme (19) mit dem wenigstens einem Zapfenvorsprung (16, 17; 22, 23) korrespondierender Hinterschneidung, insbesondere Aufnahmenut (20) einfügbar ist, wobei der wenigstens eine sich radial nach außen erstreckende Zapfenvorsprung (16, 17; 22, 23) im Abstand vom freien Zapfenende (24) ausgebildet ist,
**dadurch gekennzeichnet, daß**
wenigstens zwei Zapfenvorsprünge (16, 17; 22, 23) vorgesehen sind, die entweder diametral oder in einem vorbestimmten Winkel (α) zueinander stehen.

2. Tibiakomponente nach Anspruch 1,
**dadurch gekennzeichnet, daß**
der Zapfenvorsprung (22, 23) nasen- bzw. stiftartig ausgebildet ist (Fig. 9 bis 14).

3. Tibiakomponente nach Anspruch 1,
**dadurch gekennzeichnet, daß**
der Zapfenvorsprung (16, 17) flansch- bzw. scheibenartig ausgebildet ist (Fig. 1 bis 8).

4. Tibiakomponente nach einem der Ansprüche 1 bis 3,
**dadurch gekennzeichnet, daß**
die Zapfenaufnahme (19) im Lagerkörper (12) einen Öffnungsquerschnitt (21) aufweist, der der Projektionsfläche des Lagerzapfens (15) samt Vorsprung bzw. Vorsprüngen (16, 17; 22, 23) entspricht, so daß der Lagerzapfen (15) samt Vorsprung bzw. Vorsprüngen (16, 17; 22, 23) in vorbestimmter Rotationsstellung zwischen Lagerkörper (12) und Tibialagerfläche (11) in die Zapfenaufnahme (19) einfügbar ist, und zwar soweit, bis der bzw. die Zapfenvorsprünge sich auf Höhe der in der Zapfenaufnahme (19) ausgebildeten Hinterschneidung bzw. Zapfenvorsprung-Aufnahmenut (20) befinden und diese untergreifen bzw. in diese eingreifen können.

5. Tibiakomponente nach einem der Ansprüche 1 bis 4,
**dadurch gekennzeichnet, daß**
der bzw. die Zapfenvorsprünge als mondsichelartige Scheiben (16, 17) ausgebildet sind.

6. Tibiakomponente nach einem der Ansprüche 1 bis 5,
**dadurch gekennzeichnet, daß**
der oder die Zapfenvorsprünge (16, 17; 22, 23) auf Höhe von etwa 2/3 der Gesamthöhe des Lagerzapfens (15) ausgebildet ist bzw. sind.

7. Tibiakomponente nach einem der Ansprüche 1 bis 6,
**dadurch gekennzeichnet, daß**
dieser mit einem Lagerkörper (12) kombinierbar ist, dessen Zapfenaufnahme (19) entweder durchgehend einen Querschnitt entsprechend dem Öffnungsquerschnitt (21) der Zapfenaufnahme (19) oder einen über die Tiefe der Zapfenaufnahme (19) geteilten Querschnitt aufweist derart, daß der Querschnitt bis zur Hinterschneidung oder Zapfenvorsprung-Aufnahmenut (20) dem Öffnungsquerschnitt (21) entspricht, während der Querschnitt (25) im übrigen kreiszylindrisch ist entsprechend dem Querschnitt des kreiszylindrischen Lagerzapfens (15).

8. Tibiakomponente nach einem der Ansprüche 1 bis 7,
**dadurch gekennzeichnet, daß**
der oder die Zapfenvorsprünge (16, 17) sich in Richtung posterior/anterior erstrecken, während der korrespondierende Öffnungsquerschnitt (21) der Zapfenaufnahme (19) im Lagerkörper (12) sich in einem Winkel (β), insbesondere in einem Winkel von etwa 45° dazu erstreckt.

9. Tibiakomponente nach einem der Ansprüche 1 bis 7,
**dadurch gekennzeichnet, daß**
der oder die Zapfenvorsprünge (16, 17) sich in Richtung posterior/anterior erstrecken, während der korrespondierende Öffnungsquerschnitt (21) der Zapfenaufnahme (19) im Lagerkörper (12) sich im wesentlichen quer dazu erstreckt.

10. Tibiakomponente nach einem der Ansprüche 1 bis 9,
**dadurch gekennzeichnet, daß**
die Zapfenvorsprung-Aufnahmenut entweder der Kontur des Öffnungsquerschnitts (21) der Zapfenaufnahme (19) folgt oder alternativ kreisförmig ist mit einem Innendurchmesser, der der maximalen Erstreckung des Öffnungsquerschnitts (21) der Zapfenaufnahme (19) entspricht.

## Claims

1. Tibial component (10) of a knee joint endoprosthesis, having a flat tibial bearing surface (11), a bearing body (12) which is slidably mounted thereon and which has two concavely curved bearing shells (13) for movably accommodating a femoral component, and a rotary guide means which guides the bearing body (12) on the tibial bearing surface (11) about an axis of rotation (14) extending perpendicular thereto, the rotary guide means comprising a circular-cylindrical bearing peg (15) which stands upright on the tibial bearing surface (11) and which has at least one projection (16, 17; 22, 23) extending radially outwards, which is insertable into a peg recess (19) formed in the sliding surface (18) of the bearing body (12) - which sliding surface (18) faces the tibial bearing surface (11) - or on the underside of the bearing body and having an undercut - especially a receiving groove (20) - corresponding to the at least one peg projection (16, 17; 22, 23), the at least one peg projection (16, 17; 22, 23) which extends radially outwards being spaced away from the free end (24) of the peg,
**characterised in that**
at least two peg projections (16, 17; 22, 23) are provided, which are arranged either diametrically or at a predetermined angle (α) in relation to one another.

2. Tibial component according to claim 1,
**characterised in that**
the peg projection (22, 23) is of nose-like or pin-like construction (Figs. 9 to 14).

3. Tibial component according to claim 1,
**characterised in that**
the peg projection (16, 17) is of flange-like or disc-iike construction (Figs. 1 to 8).

4. Tibial component according to one of claims 1 to 3,
**characterised in that**
the peg recess (19) in the bearing body (12) has at its opening a cross-section (21) that corresponds to the projected area of the bearing peg (15) and projection(s) (16, 17; 22, 23), so that the bearing peg (15) and projection(s) (16, 17; 22, 23) can, in a predetermined position of rotation between the bearing body (12) and the tibial bearing surface (11), be inserted into the peg recess (19) - more specifically until the peg projection(s) are located at the level of the undercut or of the peg projection receiving groove (20), which are formed in the peg recess (19), and can engage therebehind or therein, respectively.

5. Tibial component according to one of claims 1 to 4,
**characterised in that**
the peg projection(s) are in the form of crescent-moon-like discs (16, 17).

6. Tibial component according to one of claims 1 to 5,
**characterised in that**
the peg projection(s) (16, 17; 22, 23) is/are formed at a level about 2/3 of the overall height of the bearing peg (15).

7. Tibial component according to one of claims 1 to 6,
**characterised in that**
it is combinable with a bearing body (12) wherein the peg recess (19) either has a cross-section throughout that corresponds to the cross-section (21) of the peg recess (19) at its opening or has a cross-section that is divided over the depth of the peg recess (19) in such a manner that, up to the undercut or peg projection receiving groove (20), the cross-section corresponds to the cross-section (21) at the opening whereas, for the remainder, the cross-section (25) is circular-cylindrical corresponding to the cross-section of the circular-cylindrical bearing peg (15).

8. Tibial component according to one of claims 1 to 7,
**characterised in that**
the peg projection(s) (16, 17) extend in the posterior/anterior direction, whereas the corresponding cross-section (21) at the opening of the peg recess (19) in the bearing body (12) extends at an angle (β), especially at an angle of about 45°, thereto.

9. Tibial component according to one of claims 1 to 7,
**characterised in that**
the peg projection(s) (16, 17) extend in the posterior/anterior direction, whereas the corresponding cross-section (21) at the opening of the peg recess (19) in the bearing body (12) extends in a substantially transverse direction thereto.

10. Tibial component according to one of claims 1 to 9,
**characterised in that**
the peg projection receiving groove either follows the contour of the cross-section (21) at the opening of the peg recess (19) or, alternatively, is circular with an intemal diameter that corresponds to the maximum extent of the cross-section (21) at the opening of the peg recess (19).

## Revendications

1. Élément tibial (10) pour une endoprothèse du genou comportant une surface d'appui tibiale (11) plane, un support (12) logé de manière à pouvoir glisser sur celle-ci et muni de deux coussinets (13) à courbure concave destinés à recevoir de manière mobile un élément fémoral, et comportant un guidage rotatif, par lequel le support (12) sur la surface d'appui tibiale (11) est guidé autour d'un axe de rotation (14) vertical perpendiculaire à celle-ci, le guidage rotatif comportant un tenon (15) cylindrique circulaire vertical, qui est agencé verticalement sur la surface d'appui tibiale (11) et est muni d'au moins une saillie (16, 17 ; 22, 23) s'étendant radialement vers l'extérieur, et qui est destiné à être enfiché dans une face de glissement (18) du support (12), orientée vers la surface d'appui tibiale (11), plus précisément dans un logement (19) ménagé dans ladite face de glissement (18) et comportant une contre-dépouille correspondant à ladite au moins une saillie (16, 17 ; 22, 23), en particulier une rainure de réception (20), ladite au moins une saillie (16, 17 ; 22, 23) s'étendant radialement vers l'extérieur étant réalisée à distance de l'extrémité libre du tenon (24),
**caractérisé en ce qu'**il est prévu au moins deux saillies (16, 17 ; 22, 23) qui sont agencées diamétralement l'une à l'autre ou selon un angle (α) prédéterminé.

2. Élément tibial selon la revendication 1, **caractérisé en ce que** la saillie (22, 23) est conçue en forme de bec ou de téton (figures 9 à 14).

3. Élément tibial selon la revendication 1, **caractérisé en ce que** la saillie (16, 17) est conçue en forme de collerette ou de rondelle (figures 1 à 8).

4. Élément tibial selon l'une quelconque des revendications 1 à 3, **caractérisé en ce que** le logement pour tenon (19) dans le support (12) comporte une section d'ouverture (21), qui correspond à la surface de projection du tenon (15), y compris la saillie ou les saillies (16, 17 ; 22, 23), de telle sorte que le tenon (15), y compris la saillie ou les saillies (16, 17 ; 22, 23) peut être enfiché dans le logement pour tenon (19) dans des positions de rotation prédéfinies entre le support (12) et la surface d'appui tibiale (11), à savoir à une profondeur telle que la ou les saillies sont situées à hauteur de la contre-dépouille ou rainure de réception réalisée dans le logement pour tenon (19) et qu'elles s'engagent en dessous de celle-ci ou dans celle-ci.

5. Élément tibial selon l'une quelconque des revendications 1 à 4, **caractérisé en ce que** la ou les saillies sont conçues sous forme de rondelles (16, 17) en forme de croissant de lune.

6. Élément tibial selon l'une quelconque des revendications 1 à 5, **caractérisé en ce que** la ou les saillies (16, 17 ; 22, 23) est ou sont réalisées à une hauteur égale à environ 2/3 de la hauteur totale du tenon (15).

7. Élément tibial selon l'une quelconque des revendications 1 à 6, **caractérisé en ce que** ledit élément tibial peut être combiné à un support (12), dont le logement pour tenon (19) a une section continue correspondant à la section d'ouverture (21) du logement pour tenon (19) ou une section discontinue sur la profondeur du logement pour tenon (19), de telle sorte que, jusqu'à la contre-dépouille ou à la rainure de réception (20), la section correspond à la section d'ouverture (21), alors que la section (25) de la partie restante est cylindrique circulaire en correspondant à la section du tenon (15) cylindrique circulaire.

8. Élément tibial selon l'une quelconque des revendications 1 à 7, **caractérisé en ce que** le ou les saillies (16, 17) sont orientées dans la direction postéro-antérieure, alors que la section d'ouverture (21) correspondante du logement pour tenon (19) dans le support (12) s'étend en formant un angle (β), en particulier un angle de 45° environ.

9. Élément tibial selon l'une quelconque des revendications 1 à 7, **caractérisé en ce que** le ou les saillies (16, 17) sont orientées dans la direction postéro-antérieure, alors que la section d'ouverture (21) correspondante du logement pour tenon (19) dans le support (12) s'étend sensiblement transversalement à ceux-ci.

10. Élément tibial selon l'une quelconque des revendications 1 à 9, **caractérisé en ce que** la rainure de réception suit le contour de la section d'ouverture (21) du logement pour tenon (19) ou, selon une variante, est circulaire avec un diamètre intérieur qui correspond à la dimension maximale de la section d'ouverture (21) du logement pour tenon (19).
